# EUROPÄISCHE PATENTSCHRIFT

(11) **EP 2 954 062 B1**
(45) Veröffentlichungstag und Bekanntmachung des Hinweises auf die Patenterteilung: **09.05.2018**
(21) Anmeldenummer: 14703332.8
(22) Anmeldetag: 05.02.2014
(51) Int. Cl.: C12P 19/02

(54) **VERFAHREN ZUR HERSTELLUNG VON FRUCTOSE**
PROCESS FOR PRODUCING FRUCTOSE
PROCÉDÉ POUR LA PRODUCTION DE FRUCTOSE

(30) Priorität: 06.02.2013 AT 500912013
(43) Veröffentlichungstag der Anmeldung: 16.12.2015
(73) Patentinhaber: Annikki GmbH, 8074 Raaba-Grambach (AT)
(72) Erfinder: ERTL, Ortwin, A-8076 Vasoldsberg (AT); SUT, Marta, A-8010 Graz (AT); BRANDNER, Martina, A-8074 Raaba-Grambach (AT)
(74) Vertreter: Nemec, Harald
(86) Internationale Anmeldenummer: PCT/EP2014/052230
(87) Internationale Veröffentlichungsnummer: WO 2014/122167

(56) Entgegenhaltungen:
- EP-A1- 0 028 136
- WO-A1-2013/117584
- NEUHAUSER WILFRIED ET AL: "NAD(P)H-dependent aldose reductase from the xylose-assimilating yeast Candida tenuis: Isolation, characterization and biochemical properties of the enzyme", BIOCHEMICAL JOURNAL, Bd. 326, Nr. 3, 1997, Seiten 683-692, XP002724842, ISSN: 0264-6021 in der Anmeldung erwähnt
- KIRSTEN SCHROER ET AL: "Metabolomics for biotransformations: Intracellular redox cofactor analysis and enzyme kinetics offer insight into whole cell processes", BIOTECHNOLOGY AND BIOENGINEERING, Bd. 104, Nr. 2, 1. Oktober 2009 (2009-10-01), Seiten 251-260, XP055119764, ISSN: 0006-3592, DOI: 10.1002/bit.22390
- NIDETZKY BERND ET AL: "Transient-state and steady-state kinetic studies of the mechanism of NADH-dependent aldehyde reduction catalyzed by xylose reductase from the yeast Candida tenuis", BIOCHEMISTRY, Bd. 40, Nr. 34, 28. August 2001 (2001-08-28), Seiten 10371-10381, XP002724843, ISSN: 0006-2960

## Beschreibung

Die vorliegende Erfindung betrifft ein Verfahren zur Herstellung von D-Fructose aus D-Glucose.

Für die industrielle Herstellung von D-Fructose wird bisher konventionell ein Verfahren in zwei Schritten angewandt, in dem D-Glucose durch Hydrolyse von Polysacchariden, wie z.B. Stärke, hergestellt und anschließend die Isomerisierung der auf diese Weise erhaltenen D-Glucose zu D-Fructose durchgeführt wird. Durch Isomerisierung von D-Glucose können 42% D-Fructose, 50% D-Glucose und etwa 8% Restpolysaccharide erhalten werden. Ein Problem dabei ist, dass die Isolierung reiner D-Fructose aus dieser Mischung, die Anwendung aufwendiger und kostspieliger Reinigungstechniken erfordert.

Eine Alternative zur Herstellung von D-Fructose durch Isomerisierung von D-Glucose ist eine Methode bei der in einem enzymatischen und einen chemischen Schritt D-Glucose zu D-Fructose umgewandelt wird.

Insgesamt sind eine große Zahl verschiedener Methoden zur Herstellung von D-Fructose aus D-Glucose bekannt.

So ist z.B. eine Reduktion von D-Glucoson zu D-Fructose bekannt, die in den meisten Fällen auf chemischem Weg durchgeführt wurde, wie etwa in EP1048672 beschrieben. In diesem Verfahren wird die D-Fructose durch katalytische Hydrierung einer Glucosonlösung mit hohem Trockenmassengehalt unter Einsatz von besonderen Druck- und Temperaturbedingungen hergestellt.

In US4321324 ist die Produktion von D-Glucoson aus D-Glucose in einem enzymatischen Schritt beschrieben, in dem D-Glucose durch eine Pyranose-2-oxidase zu D-Glucoson oxidiert und das entstehende Wasserstoffperoxid durch eine semipermeable Membran abgetrennt wird.

Die Reduktion von D-Glucoson zu D-Fructose auf enzymatischem Weg mit Hilfe einer Reduktase wurde z.B. im Buch "Microbial Transformation of non-steroid cyclic compounds" von Kieslich, Georg Thieme Publishers, Stuttgart 1976 vorgeschlagen und in Biochem J. 1997 Sep 15; 326 683-92 wurde beschrieben, dass eine Xylose Reduktase aus *Candida tenuis* D-Glucoson zu D-Fructose reduzieren kann.

Die Herstellung von D-Fructose durch Isomerisierung von D-Glucose in zwei Schritten (enzymatisch und chemisch) wurde z.B. in US4246347 beschrieben. Gemäß dem dort beschriebenen Verfahren wurde D-Glucose zuerst enzymatisch unter Verwendung einer Pyranose-2-oxidase zu D-Glucoson umgesetzt. Das dabei entstehende Wasserstoffperoxid wurde abgetrennt und wieder verwendet, oder durch eine Katalase abgebaut. In einem zweiten Schritt wurde entstandenes D-Glucoson durch Hydrierung zu D-Fructose umgesetzt. In diesem Verfahren wurden 2% Glucose eingesetzt und die beiden Schritte getrennt durchgeführt. Die Probleme bei den Verfahren sind der hohe Druck und die hohen Temperaturen so wie auch niedrige Konzentrationen von eingesetzten Substraten.

Bekannte Verfahren zur Herstellung/Isomerisierung von D-Fructose aus D-Glucose weisen meist verschiedene Nachteile auf. So ist beispielsweise ein effizienter Umsatz des Substrats mit hoher Selektivität meist nur unter Einsatz von hohen Drücken und Temperaturen möglich und die Bildung von verunreinigenden Nebenprodukten, die schwierig abzutrennen sind, ist nicht leicht zu vermeiden.

Es wurde nun überraschenderweise ein Verfahren gefunden, das einen effizienten Umsatz des Substrats mit hoher Selektivität und ohne Einsatz von hohen Drücken und Temperaturen ermöglicht, wobei die Bildung von verunreinigenden Nebenprodukten weitgehend vermieden werden kann, sodass die Abtrennung des Substrats vom Produkt nicht nötig ist und auf den Einsatz aufwändiger und kostspieliger Reinigungstechniken verzichten werden kann.

In einem Aspekt stellt die vorliegende Erfindung ein Verfahren zur Herstellung von D-Fructose aus D-Glucose zur Verfügung, das dadurch gekennzeichnet ist, dass in einer Ein-Topf-Reaktion
a) D-Glucose mit einer Pyranose-2-Oxidase enzymatisch zu D-Glucoson oxidiert, und
b) D-Glucoson mit einer Reduktase enzymatisch zu D-Fructose reduziert wird,
wobei insbesondere in Schritt b) ein Redox-Kofaktor eingesetzt wird.
Ein Verfahren, das durch die vorliegende Erfindung bereitgestellt wird, wird hier auch als Verfahren gemäß/nach vorliegender Erfindung bezeichnet.
Die vorliegende Erfindung betrifft also ein Verfahren zur Herstellung von D-Fructose aus D-Glucose in einer Ein-Topf-Reaktion in zwei enzymatischen Schritten:
Eine enzymatische Oxidation von D-Glucose zu D-Glucoson, gefolgt von einer enzymatischen Reduktion von D-Glucoson zu D-Fructose, die nach dem folgenden Reaktionsschema 1 verläuft: 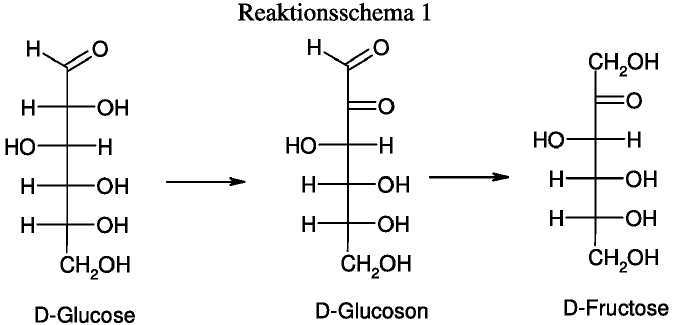 Ein Verfahren gemäß vorliegender Erfindung bietet eine neue enzymatische Möglichkeit zur Produktion von D-Fructose, ohne die Notwendigkeit zur Abtrennung und Reinigung von restlicher D-Glucose. Gegenüber aktuell angewandten Techniken stellt dabei die vorliegende Erfindung eine wesentliche Verbesserung der Verfahren zur Herstellung von D-Fructose aus D-Glucose dar. Im Gegensatz zu bestehenden Verfahren werden Verbindungen sowohl enzymatisch oxidiert als auch enzymatisch reduziert, ohne ein Zwischenprodukt isolieren zu müssen. Gleichzeitig können wesentlich höhere Substratkonzentrationen eingesetzt und auch ein höherer Umsatz erzielt werden, als das in bisher angewandten Verfahren möglich war.

Geeignete Quellen für D-Glucose in einem Verfahren nach vorliegender Beschreibung sind zum Beispiel enzymatische oder nicht-enzymatische Hydrolysate von Stärke, insbesondere Maisstärke, enzymatische oder nicht-enzymatische Hydrolysate von Saccharose oder enzymatische oder nicht-enzymatische Hydrolysate von Cellulose. Cellulose, die in einem Verfahren nach vorliegender Erfindung verwendet werden kann, kann zum Beispiel aus Biomasse gewonnen werden, vorzugsweise aus lignocellulosischer Biomasse, wie zum Beispiel Holz, Stroh, wie Weizenstroh, Maisstroh, Bagasse, Sisal, Energiegräser. Zur enzymatischen Hydrolyse von Maisstärke können z. B. Amylasen eingesetzt werden. Für die enzymatische Spaltung von Saccharose eignen sich z. B. Invertasen. Zur enzymatischen Spaltung von Cellulose können z. B. Cellulasen eingesetzt werden. Für die nicht-enzymatische Spaltung genannter Mehrfachzucker eignet sich zum Beispiel eine säurekatalysierte Spaltung.

Ein Verfahren nach vorliegender Beschreibung wird bevorzugt in einem wässrigen System durchgeführt. Es kann dem wässrigen System auch ein Puffer(system) zugesetzt werden. Geeignete Puffer(systeme) sind bekannt und schließen übliche Puffer(systeme), zum Beispiel Acetat-, Kaliumphosphat-, Tris-HCl- und Glycin-Puffer ein. Bevorzugt weist eine Puffer, der in einem Verfahren gemäß vorliegender Erfindung eingesetzt wird, einen pH-Wert von 5 bis 10,5, vorzugsweise von 6 bis 9,5 auf. Dem wässrigen System können zur Stabilisierung der Enzyme Stabilisatoren, z.B. übliche Stabilisatoren, wie etwa Ionen, z.B. Mg²⁺, oder sonstige Zusätze, z.B. übliche Zusätze, wie beispielsweise Glycerin, zugegeben werden.

In einem Verfahren nach vorliegender Beschreibung wird für die Oxidation von D-Glucose zum D-Glucoson Sauerstoff benötigt. Dieser Sauerstoff kann wie üblich eingebracht werden und z.B. durch Kontakt mit Umgebungsluft oder durch erhöhte Sauerstoffzufuhr, beispielsweise durch Druckluft oder die Einleitung von reinem Sauerstoff, zur Verfügung gestellt werden.

Ein Verfahren gemäß vorliegender Beschreibung wird bei geeigneten Temperaturen durchgeführt, die z.B. von den verwendeten Enzymen abhängig sein können. Geeignete Temperaturen schließen 10°C bis 70°C, vorzugsweise 20°C bis 50°C, z.B. 20°C bis 45°C ein Als "Ein-Topf-Reaktion" wird hier ein Verfahren bezeichnet, in dem Oxidationsreaktion und Reduktionsreaktion im selben Reaktionsansatz ohne Isolierung von Zwischenprodukten durchgeführt werden, insbesondere bei dem zwei an der Produktbildung beteiligte enzymatische Redoxreaktionen und ein enzymatisches System zur Kofaktor-Regenerierung in einem Reaktionsansatz durchgeführt werden, ohne ein Zwischenprodukt zu isolieren. Dabei können entweder alle beteiligten Enzyme gleichzeitig zugegeben werden oder es wird erst ein Teil der Enzyme zugegeben, z.B. das/die Enzym(e) für Schritt a) und mit zeitlicher Verzögerung ein anderer Teil der Enzyme, z.B. das/die Enzym(e) für Schritt b).Vor der Zugabe des zweiten Teils der Enzyme können die bereits im Reaktionsansatz vorhandenen Enzyme beispielsweise inaktiviert werden, z.B. mit einem üblichen Verfahren, wie z.B. Erhöhung der Temperatur, beispielsweise auf 65°C für 10 min.
In einem besonderen Aspekt ist ein Verfahren gemäß vorliegender Erfindung dadurch gekennzeichnet, dass das Verfahren ohne Isolierung von Zwischenprodukten erfolgt.
Die Oxidation von D-Glucose zu D-Glucoson in einem Verfahren gemäß vorliegender Beschreibung erfolgt enzymatisch, nämlich durch enzymatische Katalyse, und kann gemäß einem bekannten Verfahren durchgeführt werden. Der erfindungsgemäße Oxidationsschritt erfolgt durch Katalyse mit einer Pyranose-2-Oxidase.
Pyranose-2-oxidasen sind z.B. erhältlich aus *Coriolus sp., Aspergillus sp.* oder *Polyporus obtusus.*
Bei der Reaktion der Pyranose-2-Oxidase entsteht H₂O₂, das aus der Reaktionsmischung entfernt wird. Die Entfernung von H₂O₂ kann nach üblichen Methoden erfolgen und erfolgt bevorzugt enzymatisch, z.B. mit Hilfe einer Katalase. Beispielsweise wird eine Katalase der Reaktionsmischung zugegeben.

Eine besondere Ausführungsform des Verfahrens nach vorliegender Erfindung ist dadurch gekennzeichnet, dass entstehendes H₂O₂ mit Hilfe einer Katalase entfernt wird.
Geeignete Katalasen sind bekannt und zum Beispiel erhältlich aus *Aspergillus sp., Corynebacterium glutamicum* oder aus Rinderleber.
Die enzymatische Reduktion von D-Glucoson zu D-Fructose in einem Verfahren gemäß vorliegender Beschreibung kann nach einem geeigneten Verfahren, z.B. nach einem üblichen Verfahren, oder wie hier beschrieben erfolgen. Als Enzym zur Reduktion können geeignete, z.B. übliche Enzyme, die zur Reduktion von Substraten geeignet sind, eingesetzt werden. Geeignete Enzyme umfassen beispielsweise Reduktasen, insbesondere Xylose-Reduktasen. Geeignete Xylose-Reduktasen sind bekannt und zum Beispiel erhältlich aus *Candida tropicalis, Candida parapsilosis* oder *Debariomyces hansenii.*
Eine besondere Ausführungsform des Verfahrens nach vorliegender Erfindung ist dadurch gekennzeichnet, dass zur Reduktion von D-Glucoson zu D-Fructose eine Xylose-Reduktase eingesetzt wird.
In einem Verfahren nach vorliegender Erfindung wird ein Redox-Kofaktor, insbesondere NAD(P)H/NAD(P)⁺ eingesetzt, insbesondere wird bei der Reduktion des D-Glucoson zur D-Fructose NAD(P)H als Redox-Kofaktor eingesetzt. Dabei bezeichnet NAD⁺ die oxidierte Form und NADH die reduzierte Form von Nicotinamidadenindinucleotid, während NADP⁺ die oxidierte Form und NADPH die reduzierte Form von Nicotinamidadenindinucleotidphosphat bezeichnen. Durch die Verwendung eines Zell-Lysates des die beteiligten Enzyme exprimierenden Mikroorganismus, z.B. *E. coli,* wie z.B. *E. coli* BL21 (DE 3), in dem das benötigte NAD(P) enthalten ist, kann u.U. die kostspielige Zugabe dieses Kofaktors entfallen. Falls die Redoxkofaktoren NAD(P)⁺ und/oder NAD(P)H bei der Umsetzung von D-Glucose zu D-Fructose zugesetzt werden, beträgt die zugesetzte Konzentration in einem Verfahren gemäß vorliegender Erfindung üblicherweise von 0,001 mM bis 10 mM, vorzugsweise von 0,01 mM bis 1 mM.

Eine besondere Ausführungsform des Verfahrens nach vorliegender Erfindung ist dadurch gekennzeichnet, dass, insbesondere bei der Reduktion von D-Glucoson, Redox-Kofaktoren, insbesondere NAD(P)H, eingesetzt werden, insbesondere, dass das Enzym das im Schritt b) verwendet wird, NADP(H) abhängig ist.

Redox-Kofaktoren können durch ein geeignetes Kofaktor-Regenerierungssystem regeneriert, nämlich einem Recycling unterzogen werden, wobei die Kofaktoren wieder in die ursprünglich eingesetzte Form umgewandelt werden.

Eine besondere Ausführungsform des Verfahrens nach vorliegender Erfindung ist dadurch gekennzeichnet, dass eingesetzte Redox-Kofaktoren einem Recycling unterworfen werden, insbesondere durch ein geeignetes Kofaktor-Regenerierungssystem.

Die Regenerierung von Redox-Kofaktoren erfordert im Allgemeinen die Anwesenheit eines geeigneten Co-Substrates, das während der Regenerierung der Redox-Kofaktoren verbraucht wird. Co-Substrate, die beispielsweise bei Verwendung der Co-Faktoren NAD(P)H/NAD(P)⁺ verwendet werden können, schließen z.B. Alkohole, wie etwa Isopropylalkohol (2-Propanol, IPA), Milchsäure und deren Salze, Brenztraubensäure und deren Salze, Sauerstoff, Wasserstoff und/oder Ameisensäure und deren Salze ein.

In einem besonderen Aspekt ist ein Verfahren der vorliegenden Erfindung dadurch gekennzeichnet, dass der Redox-Kofaktor bei der Verwendung der Co-Faktoren NAD(P)H/NAD(P)⁺, insbesondere bei der Reduktion von D-Glucoson, unter Vebrauch eines Co-Substrates, insbesondere ausgewählt aus einem Alkohol, Milchsäure und deren Salze, Brenztraubensäure und deren Salze, Sauerstoff, Wasserstoff und/oder Ameisensäure und deren Salze, regeneriert wird.

Eine besondere Ausführungsform eines Verfahrens nach vorliegender Erfindung ist dadurch gekennzeichnet, dass bei der Regenerierung der Redox-Kofaktoren, insbesondere bei der Reduktion von D-Glucoson zur D-Fructose, Co-Substrate eingesetzt werden.

Bei der Regenerierung von Redox-Co-Faktoren wird ein Redoxenzym eingesetzt. Redoxenzyme, die bei der Verwendung von NAD(P)H/ NAD(P)⁺ als Redox-Co-Faktoren in Frage kommen, schließen beispielsweise Dehydrogenasen, z.B. Alkoholdehydrogenasen, Laktatdehydrogenasen, Formiatdehydrogenasen, bevorzugt Alkoholdehydrogenasen ein. Geeignete Alkoholdehydrogenasen sind bekannt und schließen z.B. eine Alkoholdehydrogenase, die aus *Lactobacillus kefir* erhältlich ist, ein.

In einer weiteren besonderen Ausführungsform des Verfahrens nach vorliegender Erfindung wird der Redox-Kofaktor durch ein Redoxenzym, insbesondere durch eine Alkoholdehydrogenase regeneriert.

Enzyme können in einem Verfahren gemäß vorliegender Erfindung als solche, gegebenenfalls in der Form von Zell-Lysaten, gegebenenfalls als rekombinant überexprimierte Proteine, beispielsweise als in *E. coli* rekombinant überexprimierte Proteine verwendet werden, wobei bevorzugt die entsprechenden Zell-Lysate ohne weitere Aufreinigung eingesetzt werden können. Je nach dem zu produzierenden Enzym können auch andere Mikroorganismen zur Expression eingesetzt werden, z.B. Mikroorganismen, die dem Fachmann bekannt sind. Feste Bestandteile der jeweiligen Mikroorganismen können in einem Verfahren gemäß vorliegender Erfindung entweder abgetrennt werden oder in der Reaktion mit eingesetzt werden (z.B. Ganzzell-Biokatalysatoren). Es können auch Kulturüberstände oder Lysate von Mikroorganismen eingesetzt werden, die ohne rekombinante DNA-Technologie bereits ausreichende Enzym-Aktivitäten aufweisen. Die Enzym-Einheit 1 U entspricht dabei derjenigen Enzymmenge, die benötigt wird, um 1 µmol Substrat pro min umzusetzen.

In einem Verfahren gemäß vorliegender Erfindung können sowohl eines oder mehrere Enzyme, als auch ein oder mehrere Redoxkofaktor(en) bei der Umwandlung von D-Glucose in D-Fructose, entweder in löslicher Form oder an Träger (Feststoffe) immobilisiert, eingesetzt werden.

In einem weiteren Aspekt ist ein Verfahren gemäß vorliegender Erfindung dadurch gekennzeichnet, dass es nach dem folgenden Reaktionsschema 2 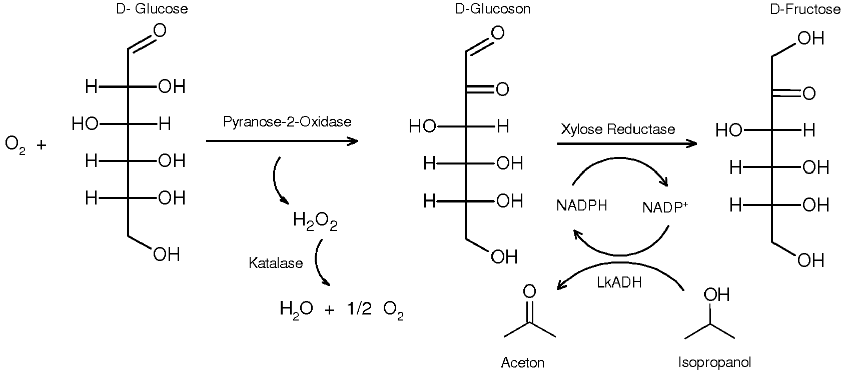 in dem LkADH eine Alkoholdehydrogenase, insbesondere eine Alkoholdehydrogenase aus *Lactobacillus kefir,* die NADP(H)-abhängig ist, bedeutet, abläuft.

D-Fructose, die gemäß vorliegender Erfindung gewonnen wurde, kann aus dem Reaktionsgemisch, z.B. nach einem üblichen Verfahren, beispielsweise mittels Kristallisation isoliert werden.

D-Fructose stellt ein wichtiges Ausgangsmaterial zur Weiterverarbeitung in der chemischen Industrie dar. Beispielsweise kann D-Fructose bekanntlich in Furanderivate, wie z.B. Hydroxymethylfurfural (HMF) der Formel 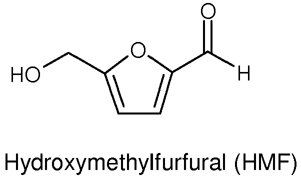 weiterverarbeitet werden.

Hydroxymethylfurfural ist bekanntlich ein Ausgangsprodukt für die Herstellung von 2,5-Furandicarbonsäure (FDCA) der Formel 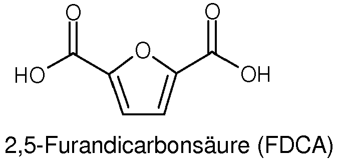 die sich bekanntlich als Monomer zur Herstellung von Polymeren wie zum Beispiel Polyethylenfuranoat (PEF) eignet. PEF kann ähnlich eingesetzt werden, wie Polyethylenterephtalat (PET), zum Beispiel zur Herstellung von Hohlkörpern, insbesondere Flaschen, wie z.B. Getränkeflaschen, Flaschen für Kosmetika oder Flaschen für Reinigungsmittel. Bei gleichzeitiger Verwendung von Ethylenglycol aus regenerativen Quellen und FDCA, welches zugänglich ist aus HMF, das in einem Verfahren gemäß vorliegender Erfindung hergestellt ist, kann PEF gewonnen werden, das vollständig aus nachwachsenden Rohstoffen besteht.

In einer besonderen Ausführungsform des Verfahrens gemäß vorliegender Beschreibung wird die hergestellte Fructose weiter zu Furanderivaten, wie z.B. Hydroxymethylfurfural (HMF) der Formel 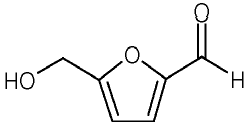 umgesetzt.
In den nachfolgenden Beispielen sind alle Temperaturangaben in Grad Cselsius (°C). Die Enzym-Einheit "1 U" entspricht dabei derjenigen Enzymmenge, die benötigt wird, um 1 µmol Substrat pro min umzusetzen
Die folgenden Abkürzungen werden verwendet:

| | |
|---|---|
| h | Stunde(n) |
| min | Minute(n) |

### Beispiel 1

### Biokonversion von D-Glucose zu D-Glucoson durch Pyranose-Oxidase unter der Verwendung von Katalase zur Entfernung des dabei gebildeten H₂O₂

Ein 0,5 ml Ansatz enthält 2,5 % (w/v) D-Glucose und 1 U Pyranose-2-Oxidase (Sigma Aldrich). Zur Umsetzung des in dieser Reaktion gebildeten H₂O₂ werden 50 U Katalase (Sigma Aldrich) eingesetzt, die das entstehende H₂O₂ zu H₂O + ½ O₂ umsetzt. Die Reaktion wird in einem Tris-HCl-Puffer (50 mM, pH 7,0) bei 30°C unter kontinuierlichem Schütteln (850 rpm) durchgeführt. Es wird ein offenes System verwendet, um eine ausreichende Sauerstoffzufuhr zu erzielen. Nach 48 h waren 99 % der D-Glucose zu D-Glucoson umgesetzt.

### Beispiel 2

### Biokonversion von D-Glucoson zu D-Fructose durch Xylose-Reduktase unter der Verwendung eines Alkoholdehydrogenase-abhängigen Kofaktor-Regenerierungssystems

Ein 0,5 ml Ansatz enthält 2,5 % (w/v) D-Glucoson und 10 U der rekombinanten Xylose-Reduktase aus *Candida tropicalis* (überexprimiert in *E. coli* BL21 (DE3)). Zur Regenerierung von NADPH werden 10 U der rekombinanten Alkoholdehydrogenase aus *Lactobacillus kefir* (überexprimiert in *E. coli* BL21 (DE3)) und anfänglich 5 % (w/v) 2-Propanol eingesetzt. Die Reaktion wird ohne Zugabe von NADPH durchgeführt. Der Kofaktor steht aus dem Zellextrakt des zur Expression der Xylose-Reduktase und der Alkoholdehydrogenase verwendeten *E. coli* BL21 (DE3) zur Verfügung. Die Reaktion wird in einem Tris-HCl-Puffer (50 mM, pH 7,0) bei 30°C und unter kontinuierlichem Schütteln (850 rpm) durchgeführt. Es wird ein offenes System verwendet, um die Verdampfung von Aceton zu ermöglichen und die Reaktion in Richtung D-Fructose zu verschieben. Es werden 2,5 % (w/v) IPA nach 6 h, 5 % IPA (w/v) nach 18 h und 2,5 % (w/v) IPA nach 24 h nachdosiert. Nach 48 h waren -90 % des D-Glucoson zu D-Fructose umgesetzt.

### Beispiel 3

### Biokonversion von D-Glucose zu D-Glucoson und weiter zu D-Fructose in einer Eintopfreaktion (zwei aufeinanderfolgende Schritte ohne Isolierung des Zwischenproduktes) unter der Verwendung eines Alkoholdehydrogenase-abhängigen Kofakor-Regenerierungssystems

Ein 0,5 ml Ansatz enthält 2,5% (w/v) D-Glucose und 1 U Pyranose-2-Oxidase (Sigma Aldrich). Zur Umsetzung des in dieser Reaktion gebildeten H₂O₂ werden 50 U Katalase eingesetzt, die das entstehende H₂O₂ zu H₂O + ½ O₂ umsetzt. Die Reaktion wird in einem Tris-HCl-Puffer (50 mM, pH 7,0) bei 30 °C unter kontinuierlichem Schütteln (850 rpm) durchgeführt. Des Weiteren wird ein offenes System verwendet, um eine ausreichende Sauerstoffzufuhr zu erzielen. Nach 24 h wird die Reaktionsmischung für 10 Minuten auf 65°C erhitzt, um die Enzyme zu deaktivieren. Anschließend werden der Reaktionsmischung 10 U der rekombinanten Xylose-Reduktase aus *Candida tropicalis* (überexprimiert in *E. coli* BL21 (DE3)) zugesetzt. Zur Regenerierung von NADPH werden 10 U der rekombinanten Alkoholdehydrogenase aus *Lactobacillus kefir* (überexprimiert in *E. coli* BL21 (DE3)) und anfänglich 5% (w/v) 2-Propanol eingesetzt. Die Reaktion wird ohne Zugabe von NADPH durchgeführt. Der Kofaktor steht aus dem Zellextrakt des zur Expression der rekombinanten Xylose-Reduktase und der rekombinanten Alkoholdehydrogenase verwendeten *E. coli* BL21 (DE3) zur Verfügung. Die Reaktion wird bei 30°C und unter kontinuierlichem Schütteln (850 rpm) durchgeführt. Es wird ein offenes System verwendet, um die Verdunstung von Aceton zu ermöglichen und die Reaktion in Richtung D-Fructose zu verschieben. Es werden 2,5% (w/v) IPA nach 6 h, 5% (w/v) IPA nach 18 h und 2,5 % (w/v) IPA nach 24 h nachdosiert. Nach 48 h waren 91 % der eingesetzten D-Glucose zu D-Fructose umgesetzt.

## Patentansprüche

1. Verfahren zur Herstellung von D-Fructose aus D-Glucose **dadurch gekennzeichnet, dass** in einer Ein-Topf-Reaktion
a) D-Glucose mit einer Pyranose-2-Oxidase enzymatisch zu D-Glucoson oxidiert, und
b) D-Glucoson mit einer Reduktase enzymatisch zu D-Fructose reduziert wird,
wobei insbesondere in Schritt b) ein Redox-Kofaktor eingesetzt wird.

2. Verfahren nach Anspruch 1, **dadurch gekennzeichnet, dass** das Verfahren ohne Isolierung von Zwischenprodukten erfolgt.

3. Verfahren nach Anspruch 1, **dadurch gekennzeichnet, dass** entstehendes H₂O₂ mit Hilfe einer Katalase entfernt wird.

4. Verfahren nach einem der Ansprüche 1 bis 3, **dadurch gekennzeichnet, dass** zur Reduktion von D-Glucoson zu D-Fructose eine Xylose-Reduktase eingesetzt wird.

5. Verfahren nach einem der Ansprüche 1 bis 4, **dadurch gekennzeichnet, dass**, insbesondere bei der Reduktion von D-Glucoson zu D-Fructose, Redox-Kofaktoren, insbesondere NAD(P)H, eingesetzt werden, insbesondere, dass das Enzym das im Schritt b) verwendet wird, NADP(H) abhängig ist.

6. Verfahren nach einem der Ansprüche 1 bis 5, **dadurch gekennzeichnet, dass** die Redox-Kofaktoren NAD(P)⁺ und/oder NAD(P)H bei der Umsetzung von D-Glucose zu D-Fructose zugesetzt werden.

7. Verfahren nach einem der Ansprüche 5 oder 6, **dadurch gekennzeichnet, dass** eingesetzte Redox-Kofaktoren einem Recycling unterworfen werden, insbesondere durch ein geeignetes Kofaktor-Regenerierungssystem.

8. Verfahren nach einem der Ansprüche 5 bis 7, **dadurch gekennzeichnet, dass** der Redox-Kofaktor bei der Verwendung der Co-Faktoren NAD(P)H/NAD(P)⁺, insbesondere bei der Reduktion von D-Glucoson zu D-Fructose, unter Vebrauch eines Co-Substrates, insbesondere ausgewählt aus einem Alkohol, Milchsäure und deren Salze, Brenztraubensäure und deren Salze, Sauerstoff, Wasserstoff und/oder Ameisensäure und deren Salze, regeneriert wird

9. Verfahren nach einem der Ansprüche 5 bis 8, worin der Redox-Kofaktor durch ein Redoxenzym, insbesondere durch eine Alkoholdehydrogenase regeneriert wird.

10. Verfahren nach einem der Ansprüche 1 bis 9, **dadurch gekennzeichnet, dass** es nach dem folgenden Reaktionsschema 2 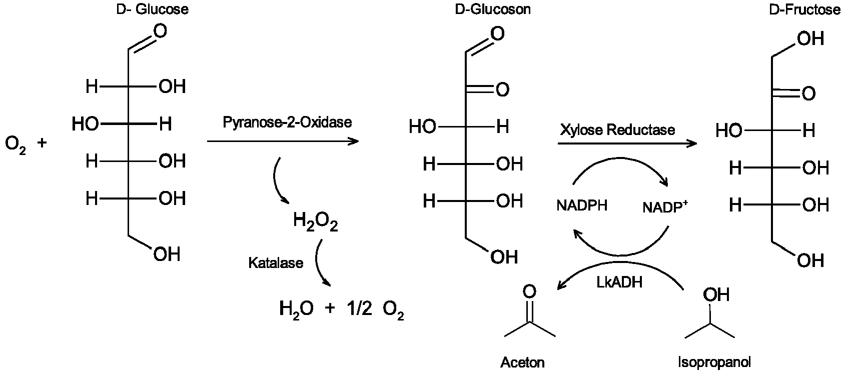 in dem LkADH eine Alkoholdehydrogenase, insbesondere eine Alkoholdehydrogenase aus *Lactobacillus kefir,* die NADP(H)-abhängig ist, bedeutet, abläuft.

11. Verfahren nach einem der Ansprüche 1 bis 10, **dadurch gekennzeichnet, dass** die hergestellte D-Fructose weiter zu Furanderivaten umgesetzt wird.

## Claims

1. A method for producing D-fructose from D-glucose, **characterized in that**, in a one-pot synthesis,
a) D-glucose is oxidized enzymatically to D-glucosone using a pyranose-2-oxidase and
b) D-glucosone is reduced enzymatically to D-fructose using a reductase,
wherein, in particular in step b), a redox cofactor will be used.

2. A method according to claim 1, **characterized in that** the method takes place without intermediates being isolated.

3. A method according to claim 1, **characterized in that** nascent H₂O₂ is removed with the aid of a catalase.

4. A method according to any one of claims 1 to 3, **characterized in that** a xylose reductase is used for the reduction of D-glucosone to D-fructose.

5. A method according to any one of claims 1 to 4, **characterized in that**, in particular during the reduction of D-glucosone to D-fructose, redox cofactors, in particular NAD(P)H, are used, in particular that the enzyme used in step b) is NADP(H)-dependent.

6. A method according to any one of claims 1 to 5, **characterized in that** the redox cofactors NAD(P)⁺ and/or NAD(P)H are added in the reaction of D-glucose to D-fructose.

7. A method according to any one of claims 5 or 6, **characterized in that** redox cofactors which are used are subjected to recycling, in particular by a suitable cofactor regeneration system.

8. A method according to any one of claims 5 to 7, **characterized in that** the redox cofactor is regenerated if the cofactors NAD(P)H/NAD(P)⁺ are used, in particular for the reduction of D-glucosone to D-fructose, consuming a cosubstrate in particular selected from an alcohol, lactic acid and salts thereof, pyruvic acid and salts thereof, oxygen, hydrogen and/or formic acid and salts thereof.

9. A method according to any one of claims 5 to 8, wherein the redox cofactor is regenerated by a redox enzyme, in particular by an alcohol dehydrogenase.

10. A method according to any one of claims 1 to 9, **characterized in that** it proceeds according to the following Reaction Scheme 2 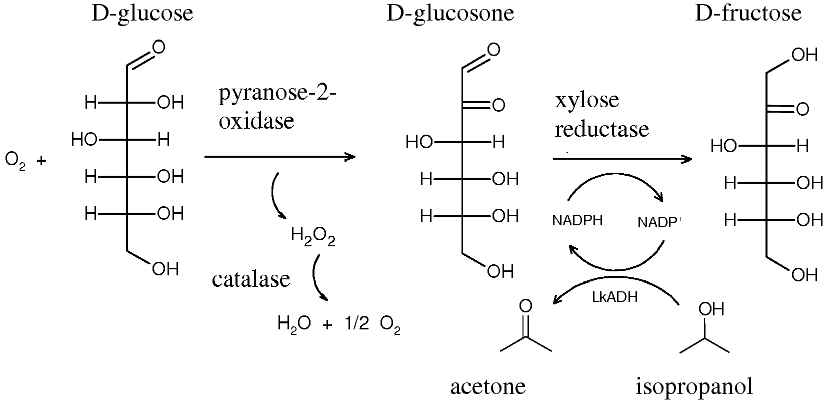 in which LkADH denotes an alcohol dehydrogenase, in particular an alcohol dehydrogenase from *Lactobacillus kefir,* which is NADP(H)-dependent.

11. A method according to any one of claims 1 to 10, **characterized in that** the produced D-fructose is converted further into furan derivatives.

## Revendications

1. Procédé de préparation de D-fructose à partir de D-glucose, **caractérisé en ce que**, dans une réaction monotope
a) on oxyde par voie enzymatique du D-glucose en une D-glucosone avec une pyranose-2-oxydase, et
b) on réduit, par voie enzymatique, de la D-glucosone en D-fructose avec une réductase,
un cofacteur redox étant utilisé, en particulier à l'étape b).

2. Procédé selon la revendication 1, **caractérisé en ce qu'**il se déroule sans isoler les produits intermédiaires.

3. Procédé selon la revendication 1, **caractérisé en ce que** le H₂O₂ qui se forme est retiré à l'aide d'une catalase.

4. Procédé selon l'une quelconque des revendications 1 à 3, **caractérisé en ce qu'**on utilise une xylose-réductase pour réduire la D-glucosone en D-fructose.

5. Procédé selon l'une quelconque des revendications 1 à 4, **caractérisé en ce que**, en particulier lors de la réduction de la D-glucosone en D-fructose, on utilise des cofacteurs redox, en particulier le NAD(P)H, en particulier **en ce que** l'enzyme utilisée à l'étape b), est NADP(H) dépendante.

6. Procédé selon l'une quelconque des revendications 1 à 5, **caractérisé en ce que** les cofacteurs redox NAD(P)⁺ et/ou NAD(P)H sont ajoutés lors de la conversion du D-glucose en D-fructose.

7. Procédé selon l'une quelconque des revendications 5 ou 6, **caractérisé en ce que** les cofacteurs redox sont soumis à un recyclage, en particulier avec un système de régénération de cofacteurs adapté.

8. Procédé selon l'une quelconque des revendications 5 à 7, **caractérisé en ce que** le cofacteur redox est régénéré lors de l'utilisation des cofacteurs NAD(P)H/NAD(P)⁺, en particulier lors de la réduction de la D-glucosone en D-fructose, en utilisant un co-substrat, en particulier choisi parmi un alcool, l'acide lactique et ses sels, l'acide pyruvique et ses sels, l'oxygène, l'hydrogène et/ou l'acide formique.

9. Procédé selon l'une quelconque des revendications 5 à 8, dans lequel le cofacteur redox est régénéré par une enzyme redox, en particulier par un alcool déshydrogénase.

10. Procédé selon l'une quelconque des revendications 1 à 9, **caractérisé en ce qu'**il se déroule selon le schéma de réaction 2 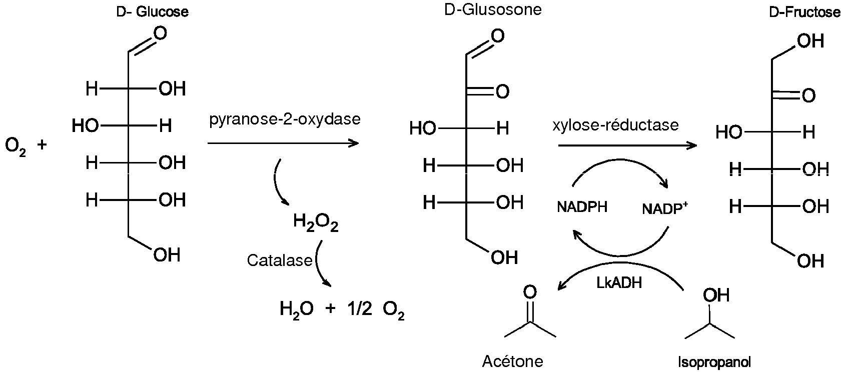 dans lequel LkADH représente un alcool déshydrogénase, en particulier un alcool déshydrogénase de *Lactobacillus kefir* qui est NADP(H) dépendante.

11. Procédé selon l'une quelconque des revendications 1 à 10, **caractérisé en ce que** le D-fructose préparé en plus est converti en dérivés de furane.
